# EUROPÄISCHE PATENTSCHRIFT

(11) **EP 0 873 341 B1**
(45) Veröffentlichungstag und Bekanntmachung des Hinweises auf die Patenterteilung: **10.09.2003**
(21) Anmeldenummer: 96939097.0
(22) Anmeldetag: 18.11.1996
(51) Int. Cl.: C07D 493/04

(54) **EPOTHILONDERIVATE, HERSTELLUNG UND VERWENDUNG**
EPOTHILONE DERIVATIVES, PREPARATION AND USE
DERIVES D'EPOTHILONE, LEUR PREPARATION ET LEUR UTILISATION

(30) Priorität: 17.11.1995 DE 19542986; 25.09.1996 DE 19639456
(43) Veröffentlichungstag der Anmeldung: 28.10.1998
(60) Teilanmeldung: 98121523.9
(73) Patentinhaber: Gesellschaft für Biotechnologische Forschung mbH (GBF), 38124 Braunschweig (DE)
(72) Erfinder: HÖFLE, Gerhard, D-38124 Braunschweig (DE); KIFFE, Michael, D-38124 Braunschweig (DE)
(74) Vertreter: Boeters, Hans Dietrich, Dr.
(86) Internationale Anmeldenummer: EP9605080
(87) Internationale Veröffentlichungsnummer: WO97019086

(56) Entgegenhaltungen:
- WO-A-93/10121

## Beschreibung

Die vorliegende Erfindung betrifft Epothilonderivate der nachfolgend dargestellten allgemeinen Formeln 3 und 6 sowie therapeutische Mittel und Mittel für den Pflanzenschutz mit diesen Epothilonderivaten.

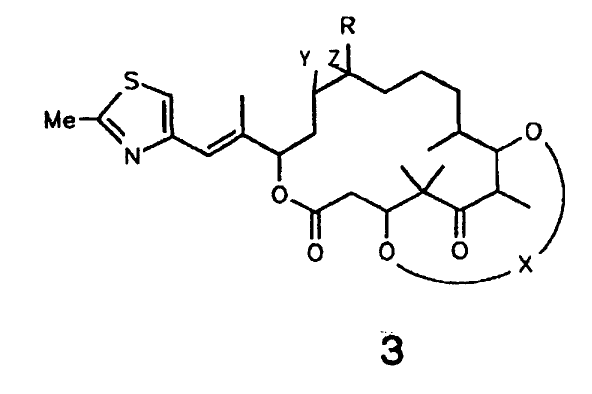

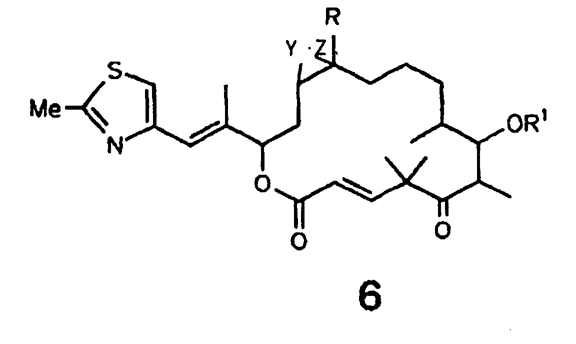

### In den vorstehenden Formeln 3 und 6 bedeuten:

- R =: H, C₁₋₄-Alkyl;
- R¹, R² =: H, C₁₋₆-Alkyl,
C₁₋₆-Acyl
Benzoyl
C₁₋₄-Trialkylsilyl,
Benzyl,
Phenyl,
C₁₋₆-Alkoxy-,
C₆-Alkyl-, Hydroxy- und Halogen-substituiertes Benzyl bzw. Phenyl;
wobei auch zwei der Reste R¹ bis R⁵ zu der Gruppierung -(CH₂)ₙmit n = 1 bis 6 zusammentreten können und es sich bei den in den Resten enthaltenen Alkyl- bzw. Acylgruppen um gradkettige oder verzweigte Reste handelt;
Y und Z sind entweder gleich oder verschieden und stehen jeweils für Wasserstoff, Halogen, wie F, Cl, Br oder J, Pseudohalogen, wie -NCO, -NCS oder -N₃, OH, O-(C₁₋₆)-Acyl, O-(C₁₋₆)-Alkyl, O-Benzoyl. Y und Z können auch das O-Atom eines Epoxides sein, wobei Epothilon A und B nicht beansprucht werden, oder eine der C-C-Bindungen einer C=C-Doppelbindung bilden.

In der **Formel 3,** steht X allgemein für -C(O)-, -C(S)-, -S(O)-, -CR¹R²- und -SiR₂-, wobei R¹ und R² die Bedeutung haben wie oben angegeben, und wobei R die Bedeutung hat wie oben angegeben.

Für Epothilon A und B sei verwiesen auf DE-A-41 38 042.

Umsetzungen von Epothilon A und B mit bifunktionellen elektrophilen Reagenzien, wie (Thio)Phosgen, (Thio)Carbonyldiimidazol, Thionylchlorid oder Dialkylsilyldichloriden bzw. -bistriflaten ergeben Verbindungen der allgemeinen **Formel 3**. Als Hilfsbasen dienen dabei Pyridin, Trialkylamine, ggf. zusammen mit DMAP bzw. 2,6-Lutidin in einem nichtprotischen Lösungsmittel. Die 3,7-Acetale der allgemeinen **Formel 3** entstehen durch Umacetalisierung z.B. von Dimethylacetalen in Gegenwart eines sauren Katalysators.

Die Verbindungen der allgemeinen **Formel 6** werden aus Derivaten von Epothilon A und B erhalten, bei denen die 7-OH-Gruppe durch Acyl- oder Ethergruppen geschützt ist, indem die 3-OH-Gruppe z.B. formyliert, mesyliert oder tosyliert und anschließend durch Behandlung mit einer Base z.B. DBU eliminiert wird. Die Freisetzung der 7-OH-Gruppen erfolgt z.B. bei O-Formyl durch NH₃/MeOH, bei O-p-Methoxybenzyl durch DDQ.

Zur Herstellung der erfindungsgemäßen Verbindungen kann man auch von Epothilon C oder D ausgehen, wobei zur Derivatisierung auf die vorstehend beschriebenen Derivatisierungsmethoden verwiesen werden kann. Dabei kann man die 12,13-Doppelbindung selektiv hydrieren, beispielsweise katalytisch oder mit Diimin; oder epoxidieren, beispielsweise mit Dimethyldioxiran oder einer Persäure; oder in die Dihalogenide, Dipseudohalogenide oder Diazide umwandeln.

Die Erfindung betrifft ferner Mittel für den Pflanzenschutz in Landwirtschaft, Forstwirtschaft und/oder Gartenbau, bestehend aus einer oder mehreren der vorstehend aufgeführten Epothilonderivate bzw. bestehend aus einem oder mehreren der vorstehend aufgeführten Epothilonderivate neben einem oder mehreren üblichen Träger(n) und/oder Verdünnungsmittel(n).

Schließlich betrifft die Erfindung therapeutische Mittel, bestehend aus einer oder mehreren der vorstehend aufgeführten Verbindungen oder einer oder mehreren der vorstehend aufgeführten Verbindungen neben einem oder mehreren üblichen Träger(n) und/oder Verdünnungsmittel(n). Diese Mittel können insbesondere cytotoxische Aktivitäten zeigen und/oder Immunsuppression bewirken und/oder zur Bekämpfung maligner Tumore eingesetzt werden, wobei sie besonders bevorzugt als Cytostatika verwendbar sind.

Die Erfindung wird im folgenden durch die Beschreibung von eini gen ausgewählten Ausführungsbeispielen näher erläutert und beschrieben.

### Beispiele

### Beispiel 1:

### Verbindung 3a-d (a-d sind Stereoisomere)

100 mg (0.203 mmol) Epothilon werden in 3 ml Pyridin gelöst, mit 50 µl (0.686 mmol) Thionylchlorid versetzt und 15 Minuten bei Raumtemperatur gerührt. Anschließend wird mit 1 M Phosphatpuffer pH 7 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Die Reinigung des Rohproduktes und Trennung der vier Stereoisomeren **3a-d** erfolgt mit Hilfe der präparativen Schichtchromatographie (Laufmittel: Toluol/Methanol, 90 : 10).

### Verbindung 3a

- Ausbeute:: 4 mg (12 %)
- R_{f} (Toluol/Methanol, 90 : 10):: 0.50
- IR (Film): ny =: 2961 (m, b, Sch), 1742 (vs), 1701 (vs), 1465 (m, Sch), 1389 (m, Sch), 1238 (s, Sch), 1210 (vs, Sch), 1011 (s, Sch), 957 (s, b, Sch), 808 (m, Sch), 768 (s, Sch) cm⁻¹
- UV (Methanol): lambdaₘₐₓ: (lg epsilon) = 210 (4.50), 248 (4.35) nm.
- MS (20/70 eV): m/e (%) =: 539 (40 [M⁺]), 457 (22), 362 (16), 316 (27), 222 (30), 178 (30), 164 (100), 151 (43), 96 (38), 69 (29), 55 (28), 43 (20).
- Hochauflösung:: C₂₆H₃₇O₇NS₂ ber.: 539.2011 für [M⁺]

### Verbindung 3b

- Ausbeute:: 14 mg (13 %)
- R_{f} (Toluol/Methanol, 90 : 10):: 0.44
- IR (Film): ny =: 2963 (s, br, Seh), 1740 (vs), 1703 (s), 1510 (w), 1464 (m, br, Sch), 1389 (m, Sch), 1240 (s, br, Sch), 1142 (m), 1076 (w), 1037 (w), 1003 (m), 945 (s, br, Sch), 806 (m, Sch), 775 (s), 737 (m) cm⁻¹.
- UV (Methanol): lambdaₘₐₓ: (lg epsilon) = 211 (4.16), 250 (4.08) nm.
- MS (20/70 eV): m/e (%) =: 539 (27 [M⁺]), 475 (17), 322 (41), 306 (67), 222 (16), 206 (17), 194 (19), 178 (32), 164 (100), 151 (33), 125 (18), 113 (15), 96 (39), 81 (23), 64 (58), 57 (42), 41 (19).
- Hochauflösung: C₂₆H₃₇O₇NS₂: ber.: 539.2011 für [M⁺]
gef.: 539.1998

### Verbindung 3c

- Ausbeute:: 4 mg (4 %)
- R_{f} (Toluol/Methanol, 90 : 10):: 0.38
- MS (20/70 eV): m/e (%) =: 539 (51 [M⁺]), 322 (22), 306 (53), 222 (36), 178 (31), 164 (100), 151 (41), 96 (25), 81 (20), 69 (26), 55 (25), 41 (25).
- Hochauflösung: C₂₆H₃₇O₇NS₂: ber.: 539.2011 für [M⁺]
gef.: 539.2001

### Verbindung 3d

- Ausbeute:: 1 mg (1 %)
- R_{f} (Toluol/Methanol, 90 : 10):: 0.33
- MS (20/70 eV) : m/e (%) =: 539 (69 [M⁺]), 322 (35), 306 (51), 222 (41), 178 (31), 164 (100), 151 (46), 96 (31), 81 (26), 69 (34), 55 (33), 41 (35)
- Hochauflösung: C₂₆H₃₇O₇NS₂: ber.: 539.2011 für [M⁺]]
gef.: 539.1997

### Beispiel 2:

### Verbindung 6a

10 mg (0.018 mmol) 3,7-Di-O-formyl-epothilon A werden in 1 ml Dichlormethan gelost, mit 27 µl (0.180 mmol)
1,8-Diazabicyclo[5.4.0]undec-7-en (DBU) versetzt und 60 Minuten bei Raumtemperatur gerührt.
Zur Aufarbeitung wird das Reaktionsgemisch mit 1 M Natriumdihydrogenphosphat-Puffer pH 4.5 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit. Nach Beseitigung des Lösungsmittel wird das resultierende Rohprodukt in 1 ml Methanol gelöst, mit 200 µl einer ammoniakalischen Methanollösung (2 mmol NH₃/ml Methanol) versetzt und über Nacht bei Raumtemperatur gerührt. Zur Aufarbeitung wird das Lösungsmittel im Vakuum entfernt.
- Ausbeute:: 4 mg (22 %)
- R_{f} (Dichlormethan/Aceton, 85 : 15):: 0.46
- IR (Film): ny =: 3445 (w, br, Sch), 2950 (vs, br, Sch), 1717 (vs, Sch), 1644 (w), 1466 (m, Sch), 1370 (m, SCh), 1267 (s, br, Sch), 1179 (s, Sch), 984 (s, Sch), 860 (w), 733 (m) cm⁻¹
- UV (Methanol) :lambdaₘₐₓ: (lg epsilon) = 210 (4.16) nm.
- MS (20/70 eV) : m/e (%) =: 475 (28 [M⁺]), 380 (21), 322 (37), 318 (40), 304 (66), 178 (31), 166 (100), 151 (29), 140 (19), 96 (38), 81 (20), 57 (26).
- Hochauflösung: C₂₆H₃₇O₅NS: ber.: 475.2392 für [M⁺]
gef. 475.2384

### Beispiel 3:

### Verbindung 6b

50 mg (0.091 mmol) 3,7-Di-O-formyl-epothilon A (werden in 1 ml Dichlorethan gelöst, mit 2 ml (0.013 mol)
1,8-Diazabicyclo[5.4.0]undec-7-en (DBU) versetzt und 12 Stunden bei 90 °C gerührt.
Zur Aufarbeitung wird das Reaktionsgemisch mit 1 M Natriumdihydrogenphosphat-Puffer pH 4.5 versetzt und die wäßrige Phase viermal mit Ethylacetat extrahiert. Die vereinigten organischen Phasen werden mit gesättigter Natriumchlorid-Lösung gewaschen, über Natriumsulfat getrocknet und vom Lösungsmittel befreit.
Die Reinigung des aus zwei Verbindungen bestehenden Rohproduktes erfolgt mittels präparativer Schichtchromatographie (Laufmittel: Dichlormethan/Aceton, 90 : 10).
- Ausbeute:: 7 mg (15 %)
Substanzcode
- R_{f} (Dichlormethan/Aceton, 90 : 10):: 0.62
- IR (Film): ny =: 2951 (m, br, Sch), 1723 (vs), 1644 (w, br, Sch), 1468 (w), 1377 (w), 1271 (m, br, Sch), 1179 (s), 987 (m, br, Seh), 735 (w, br, Sch) cm⁻¹.
- UV (Methanol): lambdaₘₐₓ: (lg epsilon) = 210 (4.44) nm.
- MS (20/70 eV) :: m/e (%) = 503 (68 [M⁺]), 408 (58), 390 (32), 334 (25), 316 (34), 220 (21), 206 (27), 194 (20), 181 (33), 164 (100), 151 (34), 139 (28), 113 (20), 96 (82), 81 (33), 67 (24), 55 (26), 43 (22).
- Hochauflösung: C₂₇H₃₇O₆NS: ber.: 503.2342 für [M⁺]
gef.: 503.2303

### Beispiel 4:

### Verbindung 6c

5 mg (0.009 mmol) 3,7-Di-O-acetyl-epothilon werden in 1 ml Methanol gelöst, mit 150 µl einer ammoniakalischen Methanollösung (2 mmol NH₃/ml Methanol) versetzt und über Nacht bei 50 °C gerührt.
Zur Aufarbeitung wird das Lösungsmittel im Vakuum entfernt. Die Reinigung des Rohproduktes erfolgt mit Hilfe der präparativen Schichtchromatographie (Laufmittel: Toluol/Methanol, 90 : 10).
- Ausbeute:: 3 mg (67 %)
- R_{f} (Dichlormethan/Aceton, 90 : 10):: 0.55
- IR (Film): ny =: 2934 (s, b, Sch), 1719 (vs, b, Sch), 1641 (m), 1460 (m, Sch), 1372 (s, Sch), 1237 (vs, b, Sch), 1179 (s, Sch), 1020 (s), 963 (s, Sch), 737 (vs) cm⁻¹.
- UV (Methanol): lambdaₘₐₓ: (lg epsilon) = 210 (4.33) nm.
- MS (20/70 eV): m/e (%) =: 517 (57 [M⁺]), 422 (58), 318 (31), 194 (20), 181 (34), 166 (100), 151 (31), 96 (96), 81 (32), 69 (27), 55 (29), 43 (69).
- Hochauflösung: C₂₈H₃₉O₆NS: ber.: 517.2498 für [M⁺]
gef.: 517 2492

### Beispiel 15:

### Epothilone C und D als Ausgangsverbindungen

### A. Produktionsstamm und Kulturbedingungen entsprechend dem Epothilon Basispatent.

### B. Produktion mit DSM 6773

75 l Kultur werden wie im Basispatent beschrieben angezogen und zum Animpfen eines Produktionsfermenters mit 700 l Produktionsmedium aus 0.8 % Stärke, 0.2 % Glukose, 0.2 % Soyamehl, 0.2 % Hefeextrakt, 0.1 % CaCl₂ x 2H₂O, 0.1 % MgSO₄ x 7H₂O, 8 mg/l Fe-EDTA, pH = 7.4 und optional 15 l Adsorberharz Amberlite XAD-16 verwendet. Die Fermentation dauert 7 - 10 Tage bei 30 C, Belüftung mit 2 m³ Luft/h. Durch Regulierung der Drehzahl wird der pO₂ bei 30 % gehalten.

### C. Isolierung

Das Adsorberharz wird mit einem 0.7 m², 100 mesh Prozeßfilter von der Kultur abgetrennt und durch Waschen mit 3 Bettvolumen Wasser/Methanol 2:1 von polaren Begleitstoffen befreit. Durch Elution mit 4 Bettvolumen Methanol wird ein Rohextrakt gewonnen, der i. Vak. bis zum Auftreten der Wasserphase eingedampft wird. Diese wird dreimal mit dem gleichen Volumen Ethylacetat extrahiert. Eindampfen der organischen Phase ergibt 240 g Rohextrakt, der zwischen Methanol und Heptan verteilt wird, um lipophile Begleitstoffe abzutrennen. Aus der Methanolphase werden durch Eindampfen i. Vak. 180 g Raffinat gewonnen, das in drei Portionen über Sephadex LH-20 (Säule 20 x 100 cm, 20 ml/min Methanol) fraktioniert wird. Die Epothilone sind in der mit 240 - 300 min Retentionszeit eluierten Fraktion von insgesamt 72 g enthalten. Zur Trennung der Epothilone wird in drei Portionen an Lichrosorb RP-18 (15 µm, Säule 10 x 40 cm, Laufmittel 180 ml/min Methanol/Wasser 65:35) chromatographiert. Nach Epothilon A und B werden mit Rₜ = 90-95 min Epothilon C und 100-110 min Epothilon D eluiert und nach Eindampfen i. Vak. in einer Ausbeute von jeweils 0.3 g als farblose Öle gewonnen.

### D. Physikalische Eigenschaften

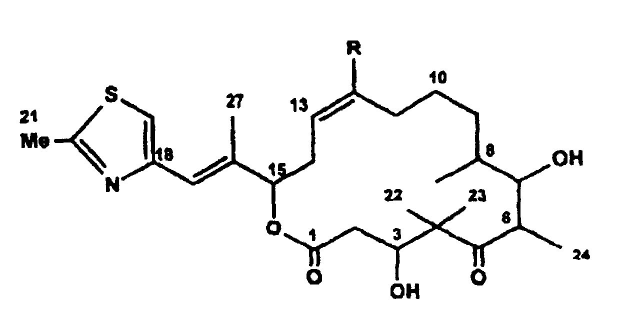

Epothilon C R = H
Epothilon D R = CH₃
**Epothilon C**
   C₂₆H₃₉NO₅S [477]
   ESI-MS: (positiv Ionen): 478.5 für [M+H]⁺
   1H und 13C siehe NMR-Tabelle
   DC:R_{f} = 0,82
   DC-Alufolie 60 F 254 Merck, Laufmittel: Dichlormethan/Methanol = 9:1
   Detektion: UV-Löschung bei 254 nm. Ansprühen mit Vanillin-Schwefelsäure-Reagenz, blau-graue Anfärbung beim Erhitzen auf 120 °C.
   HPLC:Rₜ = 11,5 min
   Säule: Nucleosil 100 C-18 7µm, 125 x 4 mm
   Laufmittel: Methanol/Wasser = 65:35
   Fluß: 1ml/min
   Detection: Diodenarray
**Epothilon D**
   C₂₇H₄₁NO₅S [491]
   ESI-MS: (positiv Ionen): 492,5 für [M+H]⁺
   1H und 13C siehe NMR-Tabelle
   DC:R_{f} = 0,82
   DC-Alufolie 60 F 254 Merck, Laufmittel: Dichlormethan/Methanol = 9:1
   Detektion: UV-Löschung bei 254 nm. Ansprühen mit Vanillin-Schwefelsäure-Reagenz, blau-graue Anfärbung beim Erhitzen auf 120 °C.
   HPLC:Rₜ = 15,3 min
   Säule: Nucleosil 100 C-18 7µm, 125 x 4 mm
   Laufmittel: Methanol/Wasser = 65:35
   Fluß: 1ml/min
   Detection: Diodenarray

**Tabelle:**

| ¹H-und ¹³C-NMR Daten von Epothilon C und Rpothilon D in [D₆]DMSO bei 300 MHz | | | | | | |
|---|---|---|---|---|---|---|
| | Epothilon C | | | Epothilon D | | |
| H-Atom | δ (ppm) | C-Atom | δ (ppm) | δ (ppm) | C-Atom | δ (ppm) |
| | | 1 | 170.3 | | 1 | 170.1 |
| 2-Ha | 2.38 | 2 | 38.4 | 2.35 | 2 | 39.0 |
| 2-Hb | 2.50 | 3 | 71.2 | 2.38 | 3 | 70.8 |
| 3-H | 3.97 | 4 | 53.1 | 4.10 | 4 | 53.2 |
| 3-OH | 5.12 | 5 | 217.1 | 5.08 | 5 | 217.4 |
| 6-H | 3.07 | 6 | 45.4 | 3.11 | 6 | 44.4 |
| 7-H | 3.49 | 7 | 75.9 | 3.48 | 7 | 75.5 |
| 7-OH | 4.46 | 8 | 35.4 | 4.46 | 8 | 36.3 |
| 8-H | 1.34 | 9 | 27.6 | 1.29 | 9 | 29.9 |
| 9-Ha | 1.15 | 10 | 30.0 | 1.14 | 10 | 25.9 |
| 9-Hb | 1.40 | 11 | 27.6 | 1.38 | 11 | 31.8* |
| 10-Ha | 1.15* | 12 | 124.6 | 1.14* | 12 | 138.3 |
| 10-Hb | 1.35* | 13 | 133.1 | 1.35* | 13 | 120.3 |
| 11-Ha | 1.90 | 14 | 31.1 | 1.75 | 14 | 31.6* |
| 11-Hb | 2.18 | 15 | 76.3 | 2.10 | 15 | 76.6 |
| 12-H | 5.38** | 16 | 137.3 | | 16 | 137.2 |
| 13-H | 5.44** | 17 | 119.1 | 5.08 | 17 | 119.2 |
| 14-Ha | 2.35 | 18 | 152.1 | 2.30 | 18 | 152.1 |
| 14-Hb | 2.70 | 19 | 117.7 | 2.65 | 19 | 117.7 |
| 15-H | 5.27 | 20 | 164.2 | 5.29 | 20 | 164.3 |
| 17-H | 6.50 | 21 | 18.8 | 6.51 | 21 | 18.9 |
| 19-H | 7.35 | 22 | 20.8 | 7.35 | 22 | 19.7 |
| 21-H₃ | 2.65 | 23 | 22.6 | 2.65 | 23 | 22.5 |
| 22-H₃ | 0.94 | 24 | 16.7 | 0.90 | 24 | 16.4 |
| 23-H₃ | 1.21 | 25 | 18.4 | 1.19 | 25 | 18.4 |
| 24-H₃ | 1.06 | 27 | 14.2 | 1.07 | 26 | 22.9 |
| 25-H₃ | 0.90 | | | 0.91 | 27 | 14.1 |
| 26-H₃ | | | | 1.63 | | |
| 27-H₃ | 2.10 | | | 2.11 | | |

| | | | | | | |
|---|---|---|---|---|---|---|
| *, ** Zuordnung vertauschbar | | | | | | |

## Patentansprüche

1. Epothilonderivat der Formel 3 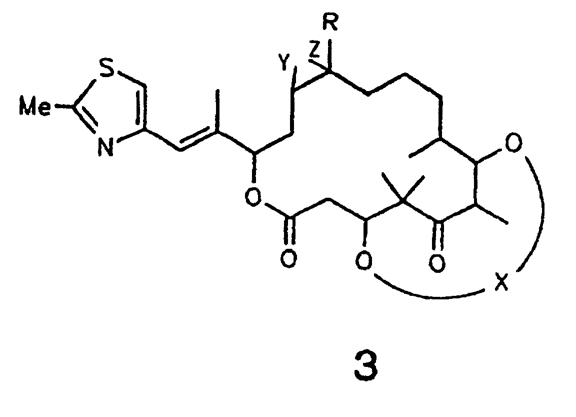 wobei R = H, C₁₋₄-Alkyl; R¹, R² = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl; es sich bei den in den Resten enthaltenen Alkyl- bzw. Acylgruppen um gradkettige oder verzweigte Reste handelt, und X allgemein für -C(O)-, -C(S)-, -S(O)-, -CR¹R²- und -SiR₂- steht, wobei R, R¹ und R² die Bedeutung haben wie oben angegeben und R¹ und R² auch zusammen eine Alkylengruppe mit 2 bis 6 Kohlenstoffatomen bilden können; und Y und Z
entweder gleich oder verschieden sind und jeweils für Wasserstoff, Halogen, Pseudohalogen, OH, O-(C₁₋₆)-Acyl, O-(C₁₋₆)-Alkyl oder O-Benzoyl stehen oder gemeinsam das O-Atom eines Epoxids oder eine der C-C-Bindungen einer C=C-Doppelbindung bilden.

2. Epothilonderivat der Formel 6 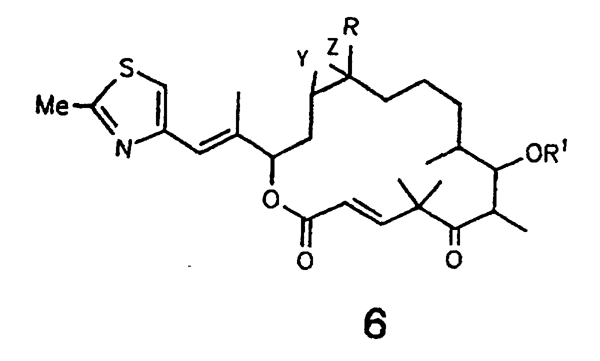 wobei R = H, C₁₋₄-Alkyl und R¹ = H, C₁₋₆-Alkyl, C₁₋₆-Acyl, Benzoyl, C₁₋₄-Trialkylsilyl, Benzyl, Phenyl, C₁₋₆-Alkoxy-, C₆-Alkyl-, Hydroxy- und halogensubstituiertes Benzyl bzw. Phenyl ist; es sich bei den in den Resten enthaltenen Alkyl- bzw. Acylgruppen um gradkettige oder verzweigte Reste handelt; und Y und Z
entweder gleich oder verschieden sind und jeweils für Wasserstoff, Halogen, Pseudohalogen, OH, O-(C₁₋₆)-Acyl, O-(C₁₋ ₆)-Alkyl oder O-Benzoyl stehen oder gemeinsam das O-Atom eines Epoxids oder eine der C-C-Bindungen einer C=C-Doppelbindung bilden.

3. Mittel für den Pflanzenschutz in der Landwirtschaft und Forstwirtschaft und/oder im Gartenbau, bestehend aus einem oder mehreren der Verbindungen gemäß einem der vorangehenden Ansprüche oder einer oder mehreren dieser Verbindungen neben einem oder mehreren üblichen Träger(n) und/oder Verdünnungsmittel(n).

4. Therapeutisches Mittel, insbesondere zum Einsatz als Cytostatikum, bestehend aus einer oder mehrerer der Verbindungen nach einem oder mehreren der Ansprüche 1 bis 2 oder einer oder mehrerer der Verbindungen nach einem oder mehreren der Ansprüche 1 bis 2 neben einem oder mehreren üblichen Träger(n) und/oder Verdünnungsmittel(n).

## Claims

1. Epothilone derivative of formula 3 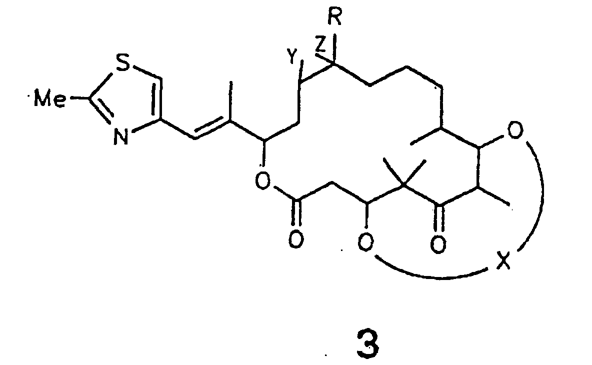 wherein
R = H or C₁₋₄alkyl;
R¹ and R² = H, C₁₋₆alkyl, C₁₋₆acyl, benzoyl, C₁₋₄trialkylsilyl, benzyl, phenyl, or benzyl or phenyl each substituted by C₁₋₆alkoxy, C₆alkyl, hydroxy or by halogen; the alkyl and acyl groups contained in the radicals are straight-chain or branched radicals; and
X generally represents -C(O)-, -C(S)-, -S(O)-, -CR¹R²- and -SiR₂-, wherein R, R¹ and R² are as defined above and
R¹ and R² may also together form an alkylene group having from 2 to 6 carbon atoms; and
Y and Z are either identical or different and each represents hydrogen, halogen, pseudohalogen, OH, O-(C₁₋₆)alkyl,
O-(C₁₋₆)acyl or O-benzoyl, together form the O atom of an epoxy group or one of the C-C bonds of a C=C double bond.

2. Epothilone derivative of formula 6 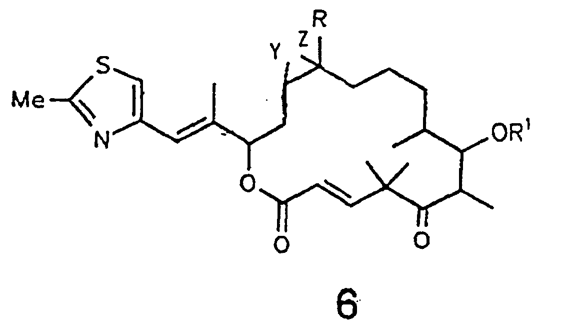 wherein
R = H or C₁₋₄alkyl;
R¹ = H, C₁₋₆alkyl, C₁₋₆acyl, benzoyl, C₁₋₄trialkylsilyl, benzyl, phenyl, or benzyl or phenyl each substituted by C₁₋₆alkoxy, C₆alkyl, hydroxy or by halogen; and the alkyl and acyl groups contained in the radicals are straight-chain or branched radicals; and
Y and Z are either identical or different and each represents hydrogen, halogen, pseudohalogen, OH, O-(C₁₋₆)alkyl,
O-(C₁₋₆)acyl or O-benzoyl, together form the O atom of an epoxy group or one of the C-C bonds of a C=C double bond.

3. Composition for plant protection in agriculture and forestry and/or in horticulture, consisting of one or more of the compounds according to any of the preceding claims or consisting of one or more of these compounds together with one or more common carrier(s) and/or diluent(s).

4. Therapeutic composition, especially for use as a cystostatic agent, consisting of one or more of the compounds according to one of more of claims 1 to 2 or consisting of one or more of the compounds according to one or more of claim 1 to 2 together with one or more common carrier(s) and/or diluent(s).

## Revendications

1. Dérivé d'épothilone, de formule 3 : 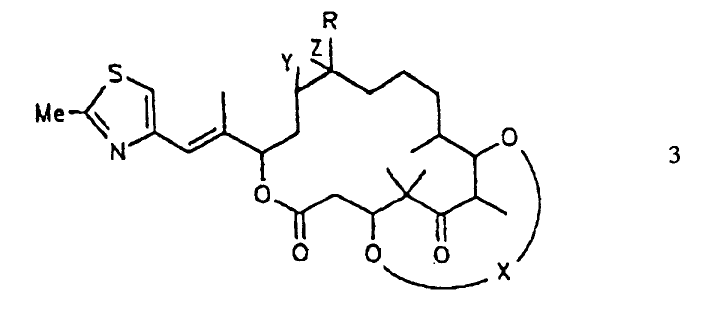 dans laquelle :
R représente un atome d'hydrogène ou un groupe alkyle en C₁₋₄ ;
R¹ et R² représentent chacun un atome d'hydrogène, ou un groupe alkyle en C₁₋₆, acyle en C₁₋₆, benzoyle, tri(alkyle en C₁₋₄)silyle, benzyle ou phényle, ou encore un groupe benzyle ou phényle à substituant(s) alcoxy en C₁₋₆, alkyle en C₁₋₆, hydroxy ou halogéno, les groupes alkyle ou acyle contenus dans les divers restes étant des groupes à chaîne linéaire ou ramifiée ;
X représente en général -C(O)-, -C(S)-, -S(O)-, -CR¹R²- ou -SiR₂-, où R, R¹ et R² ont les significations indiquées ci-dessus, R¹ et R² pouvant aussi former ensemble un groupe alkylène comportant de 2 à 6 atomes de carbone ;
et Y et Z sont identiques ou différents et représentent chacun un atome d'hydrogène ou d'halogène, un pseudohalogène, ou un groupe OH, O-acyle en C₁₋₆, O-alkyle en C₁₋₆ ou O-benzoyle, ou bien ils représentent ensemble l'atome d'oxygène d'un époxyde ou l'une des liaisons C-C d'une double liaison C=C. >

2. Dérivé d'épothilone, de formule 6 : 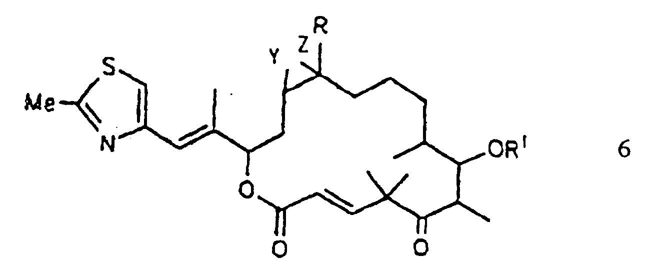 dans laquelle :
R représente un atome d'hydrogène ou un groupe alkyle en C₁₋₄ ;
R¹ représente un atome d'hydrogène, ou un groupe alkyle en C₁₋₆, acyle en C₁₋₆, benzoyle, tri(alkyle en C₁₋₄)silyle, benzyle ou phényle, ou encore un groupe benzyle ou phényle à substituant(s) alcoxy en C₁₋₆, alkyle en C₁₋₆, hydroxy ou halogéno, les groupes alkyle ou acyle contenus dans les divers restes étant des groupes à chaîne linéaire ou ramifiée ;
et Y et Z sont identiques ou différents et représentent chacun un atome d'hydrogène ou d'halogène, un pseudohalogène, ou un groupe OH, O-acyle en C₁₋₆, O-alkyle en C₁₋₆ ou O-benzoyle, ou bien ils représentent ensemble l'atome d'oxygène d'un époxyde ou l'une des liaisons C-C d'une double liaison C=C.

3. Agent de protection des végétaux, utilisable en agriculture, en sylviculture et/ou en horticulture, constitué de l'un ou de plusieurs des composés conformes à l'une des revendications précédentes, ou de l'un ou de plusieurs de ces composés en association avec un ou plusieurs véhicules et/ou diluants habituels.

4. Agent thérapeutique, destiné en particulier à être utilisé comme agent cytostatique, constitué de l'un ou de plusieurs des composés conformes à l'une ou plusieurs des revendications 1 et 2, ou de l'un ou de plusieurs des composés conformes à l'une ou plusieurs des revendications 1 et 2 en association avec un ou plusieurs véhicules et/ou diluants habituels.
